# EUROPEAN PATENT APPLICATION

(11) **EP 4 260 797 A1**
(43) Date of publication of application: **18.10.2023**
(21) Application number: 21721813.0
(22) Date of filing: 05.02.2021
(51) Int. Cl.: A61B 5/00, A61B 5/318

(54) **ELECTROCARDIOGRAPHIC SIGNAL DETECTION METHOD AND DEVICE**

(30) Priority: 10.12.2020 CN 202011434788
(71) Applicant: QINGDAO DAAI MATRIX HEALTH TECHNOLOGY CO. LTD., Qingdao Shandong 266000 (CN)
(72) Inventor: LI, Xiaoyu, Qingdao, Shandong 266000 (CN); XU, Yan, Qingdao, Shandong 266000 (CN); WANG, Zhong, Qingdao, Shandong 266000 (CN); LV, Zhixin, Qingdao, Shandong 266000 (CN)
(74) Representative: LKGLOBAL Lorenz & Kopf Patentanwalt Attorney at Law PartG mbB
(86) International application number: PCT/CN2021/075375
(87) International publication number: WO 2022/121107

(57) **Abstract**

A method and an apparatus for detecting an ECG signal. A target feature corresponding to an ECG signal is determined in a case that the ECG signal is acquired and the ECG signal is at a stable state. At least one prediction model is called to analyze the target feature, such that a signal-analysis result outputted by the called prediction model is acquired. The signal-analysis result is at least configured to identify whether the ECG signal is normal or abnormal. Hence, when applying such technical solution, stability is determined before the ECG signal is subject to actual prediction, which can eliminate an error in a calculating result due to signal instability. When the at least one prediction model is called to analyze the target feature, another prediction model is further called to analyze the target feature in a case that the signal-analysis result outputted by called prediction model(s) is in the ambiguity interval. Thereby, at least two prediction models can be applied to analyze the target feature in a case that reliability of the signal-analysis result outputted by a prediction model is low. Accuracy of the analysis is improved.

## Description

The present application claims the priority to Chinese Patent Application No. 202011434788.9, titled "METHOD AND APPARATUS FOR DETECTING ECG SIGNAL", filed on December 10, 2020 with the China National Intellectual Property Administration, which is incorporated herein by reference in its entirety.

### FIELD

The present disclosure relates to the technical field of signal processing, and in particular to a method and an apparatus for detecting an electrocardiogram (ECG) signal.

### BACKGROUND

An electrocardiogram (ECG) machine is capable to collect an ECG signal generated by myocardium excitation during heart activities, and thereby predicts a state of the heart activities through the ECG signal.

Currently, the ECG signals are generally collected to learn the heart activity. During the collection, the collected ECG signal is inaccurate due to collecting electrodes or other reasons, which further renders the prediction on the heart activities inaccurate.

Thus, how to improve accuracy of detecting the ECG signal is an urgent problem to be solved by those skilled in the art.

### SUMMARY

An objective of the present disclosure is to provide a method and an apparatus for detecting an electrocardiogram (ECG) signal, which improves accuracy of detecting the ECG signal.

In order to address the aforementioned technical issue, a method for detecting an ECG signal is provided according to embodiments of the present disclosure. The method includes:
acquiring an ECG signal;
determining a target feature corresponding to the ECG signal in a case that the ECG signal is at a stable state;
analyzing the target feature through calling at least one prediction model, to acquire a signal-analysis result outputted by the at least one prediction model, and analyzing the target feature through calling another prediction model to acquire another signal-analysis result, in a case that the signal-analysis result outputted by the at least one prediction model is in an ambiguity interval; and
acquiring an analysis result of the ECG signal according to the signal-analysis result outputted by the one or more called prediction models, where the analysis result is at least configured to identify whether the ECG signal is normal or abnormal.

In a preferable embodiment, the prediction models includes a main prediction model and at least one auxiliary prediction model, where the main prediction model is more accurate than the auxiliary prediction model; and
analyzing the target feature through calling at least one prediction model, to acquire the signal-analysis result outputted by the at least one prediction model, and analyzing the target feature through calling another prediction model to acquire another signal-analysis result, in the case that the signal-analysis result outputted by the at least one prediction model is in the ambiguity interval, includes:
analyzing the target feature through calling the main prediction model, to acquire a main signal-analysis result outputted by the main prediction model;
forbidding analyzing the target feature through calling the auxiliary prediction model, in a case that the main signal-analysis result is not in the ambiguity interval; and
analyzing the target feature through calling the at least one auxiliary prediction model, to acquire an auxiliary signal-analysis result outputted by the at least one auxiliary prediction model, in a case that the main signal-analysis result is in the ambiguity interval.

In a preferable embodiment, acquiring the analysis result of the ECG signal according to the signal-analysis result outputted by the one or more called prediction models, includes:
determining the main signal-analysis result to be the analysis result, in a case that the main signal-analysis result is not in the ambiguity interval; and
obtaining the analysis result through weighting the main signal-analysis result and the auxiliary signal-analysis result, in a case that the main signal-analysis result is in the ambiguity interval.

In a preferable embodiment, whether the ECG signal is at the stable state is determined by:
acquiring, from the ECG signal within N cycles, one or both of fluctuations among peaks and fluctuations among valleys in one or more component waves of a same type, where N is a positive integer;
determining that the ECG signal is at the stable state, in a case that the currently acquired fluctuations meet a preset condition; and
determining that the ECG signal is not at the stable state, in a case that the currently acquired fluctuations do not meet the preset condition.

In a preferable embodiment, determining the target feature corresponding to the ECG signal includes:
extracting signals of leads V2 to V5 from the ECG signal; and
extracting the target feature from the signals of leads V2 to V5.

In a preferable embodiment, extracting the target feature from the signals of leads V2 to V5 includes:
acquiring features from the signals of leads V2 to V5;
acquiring heat values corresponding to the features; and
selecting the target feature from the features based on the heat values;
where the features include amplitude, a parameter of amplitude variation, an energy spectral density, and a moment corresponding to maximum amplitude, of each component wave.

In order to address the aforementioned technical issue, an apparatus for detecting an ECG signal is further provided according to embodiments of the present disclosure. The apparatus includes:
an acquiring module, configured to acquire an ECG signal;
a determining module, configured to determine a target feature corresponding to the ECG signal in a case that the ECG signal is at a stable state;
a calling module, configured to: analyze the target feature through calling at least one prediction model, to acquire a signal-analysis result outputted by the at least one prediction model; and analyze the target feature through calling another prediction model to acquire another signal-analysis result, in a case that the signal-analysis result outputted by the at least one prediction model is in an ambiguity interval; and
an analyzing module, configured to acquire an analysis result of the ECG signal according to the signal-analysis result outputted by the one or more called prediction models, where the analysis result is at least configured to identify whether the ECG signal is normal or abnormal.

In a preferable embodiment, the prediction models includes a main prediction model and at least one auxiliary prediction model, where the main prediction model is more accurate than the auxiliary prediction model;
the calling module includes:
a calling unit, configured to analyze the target feature through calling the main prediction model, to acquire a main signal-analysis result outputted by the main prediction model; and
a controlling unit, configured to: forbid analyzing the target feature through calling the auxiliary prediction model, in a case that the main signal-analysis result is not in the ambiguity interval; and analyze the target feature through calling the at least one auxiliary prediction model, to acquire an auxiliary signal-analysis result outputted by the at least one auxiliary prediction model, in a case that the main signal-analysis result is in the ambiguity interval.

In a preferable embodiment, the analyzing module includes:
a first analyzing unit, configured to determine the main signal-analysis result to be the analysis result, in a case that the main signal-analysis result is not in the ambiguity interval; and
a second analyzing unit, configured to obtain the analysis result through weighting the main signal-analysis result and the auxiliary signal-analysis result, in a case that the main signal-analysis result is in the ambiguity interval.

In a preferable embodiment, the apparatus further includes:
a stability-determining module, configured to: acquire, from the ECG signal within N cycles, one or both of fluctuations among peaks and fluctuations among valleys in one or more component waves of a same type, where N is a positive integer; determine that the ECG signal is at the stable state, in a case that the currently acquired fluctuations meet a preset condition; and determine that the ECG signal is not at the stable state, in a case that the currently acquired fluctuations do not meet the preset condition.

In a preferable embodiment, the determining module includes:
a first extracting unit, configured to extract signals of leads V2 to V5 from the ECG signal; and
a second extracting unit, configured to extract the target feature from the signals of leads V2 to V5.

In a preferable embodiment, the second extracting unit includes:
a first acquiring sub-unit, configured to acquire features from the signals of leads V2 to V5;
a second acquiring sub-unit, configured to acquire heat values corresponding to the features; and
a selecting sub-unit, configured to select the target feature from the features based on the heat values;
where the features include amplitude, a parameter of amplitude variation, an energy spectral density, and a moment corresponding to maximum amplitude, of each component wave.

In order to address the aforementioned technical issue, an apparatus for detecting an ECG signal is further provided according to embodiments of the present disclosure. The apparatus includes:
a memory, configured to store a computer program; and
a processor, configured to implement the aforementioned method when executing the computer program.

In order to address the aforementioned technical issue, a computer-readable storage medium is further provided according to embodiments of the present disclosure. The computer-readable storage medium stores a computer program, where the computer program when executed by the processor implements the aforementioned method.

The method for detecting the ECG signal according to embodiments of the present disclosure includes following steps. The target feature corresponding to the ECG signal is determined in a case that the ECG signal is acquired and the ECG signal is at the stable state. The at least one prediction model is called to analyze the target feature, such that the signal-analysis result outputted by the called prediction model is acquired. The signal-analysis result is at least configured to identify whether the ECG signal is normal or abnormal. Hence, when applying such technical solution, stability is determined before the ECG signal is subject to actual prediction, which can eliminate an error in a calculating result due to signal instability. When the at least one prediction model is called to analyze the target feature, another prediction model is further called to analyze the target feature in a case that the signal-analysis result outputted by called prediction model(s) is in the ambiguity interval. Thereby, at least two prediction models can be applied to analyze the target feature in a case that reliability of the signal-analysis result outputted by a prediction model is low. Accuracy of the analysis is improved.

In addition, the apparatus according to embodiments of the present disclosure corresponds to the foregoing method and has similar effects.

### BRIEF DESCRIPTION OF THE DRAWINGS

For clearer illustration of the technical solutions according to embodiments of the present disclosure or conventional techniques, hereinafter briefly described are the drawings to be applied in embodiments of the present disclosure or conventional techniques. Apparently, the drawings in the following descriptions are only some embodiments of the present disclosure, and other drawings may be obtained by those skilled in the art based on the provided drawings without creative efforts.
Figure 1 is architecture of a system for detecting an ECG signal according to an embodiment of the present disclosure;
Figure 2 is a flow chart of a method for detecting an ECG signal according to an embodiment of the present disclosure;
Figure 3 is a flow chart of another method for detecting an ECG signal according to an embodiment of the present disclosure;
Figure 4 is a schematic structural diagram of an apparatus for detecting an ECG signal according to an embodiment of the present disclosure, and
Figure 5 is a schematic structural diagram of another apparatus for detecting an ECG signal according to an embodiment of the present disclosure.

### DETAILED DESCRIPTION OF EMBODIMENTS

An ECG machine is capable to collect an ECG signal. Professional technicians need to analyze whether the corresponding ECG signal is in a normal state, and such manual analysis are partially subjective. Especially, reliability of detecting the ECG signal is low when a result is in a critical range (ambiguity interval) between being normal and being abnormal. In addition, accuracy of the detection result is low in a case that the collected ECG signal is unstable.

In view of the above issue in conventional technology, a method and an apparatus for detecting an ECG signal is provided according to embodiments of the present disclosure. The technical solution collects an ECG signal, analyzes the ECG signal mathematically, and thereby obtains a result. Thus, the issue of low reliability due to subjectivity is avoided. Meanwhile, stability of the ECG signal is determined before the analysis, and a target feature is analyzed through multiple prediction models when the result is in the ambiguity interval. Hence, accuracy of the analysis result is improved.

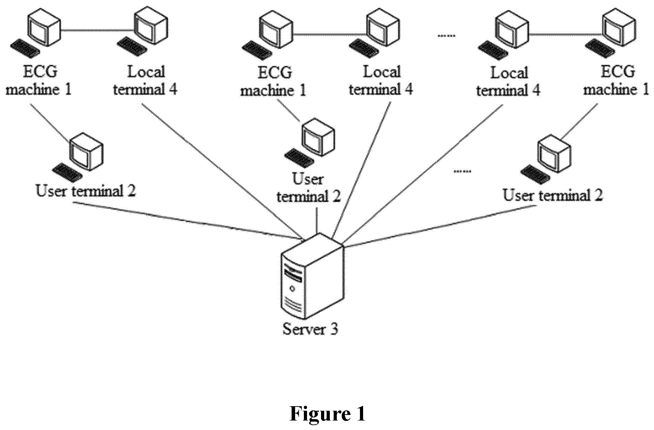

In order to facilitate understanding, hereinafter system architecture applicable to technical solutions of the present disclosure is described. Reference is made to Figure 1, which is architecture of a system for detecting an ECG signal according to an embodiment of the present disclosure.

As shown in Figure 1, a system for detecting an ECG signal according to an embodiment of the present disclosure may include ECG machines 1, user terminals 2, a server 3, and local terminals 4.

In a specific implementation, the ECG machine 1 is configured to collect an ECG signal, and includes electrodes and a host. The electrodes are fixedly attached to a corresponding part of a body under test, and are mainly configured to collect the ECG signal of the part under test and transmit the ECG signal to the host. An ECG vector generated during depolarization and repolarization of the heart is transmitted to all parts of the body through volume conduction, and an electrical potential difference is generated. An ECG graph can be depicted when two electrodes are disposed at any two points of the body under test and are connected to the host. Such manner of disposing the electrodes and connecting the electrodes with the host is called an ECG lead. The lead in embodiments of the present disclosure may be a standard lead, also called a bipolar limb lead, which reflects the electrical potential difference between two limbs. It is appreciated that the lead may be a non-standard lead besides the standard lead, which does not affect implementation of the technical solutions of the present disclosure. The host mainly includes an AD conversion circuit, a filter circuit, and a wireless communication module. The AD conversion circuit is configured to convert an analog signal into an electrical signal. The filter circuit is configured to filter the ECG signal to reduce interference. The wireless communication module includes a Bluetooth module and/or a Wi-Fi module, etc., and is configured to connect the user terminal 2 or another terminal wirelessly, so as to transmit the processed ECG signal to the user terminal 2 or another terminal.

The user terminal 2 is a link for achieving communications between the ECG machine 1 and the server 3. The user terminal 2 needs to be authenticated by the server 3. In a specific embodiment, a corresponding application (APP) may be downloaded on the user terminal to complete user registration. During operation, the user terminal 2 uploads the ECG signal acquired by the ECG machine, so that the server 3 may receive and analyze the signal. The user terminal 2 in embodiments of the present disclosure includes, but is not limited to, smart phones, tablets, desktops, and wearable devices, on which the aforementioned APP is installed.

The server 3 is configured to connect the user terminal 2 wirelessly, and the wireless connection may be specifically implemented by Wi-Fi, 4G, 5G, etc. After receiving the ECG signal uploaded by the user terminal 2, the server 3 analyzes the ECG signal to acquire a corresponding result. The server 3 can transmit the result to the user terminal 2 for the user to view.

The local terminal 4 may be connected with the server 3. The local terminal 4 is configured to acquire the ECG signal transmitted by the server, and store the ECG signal for the staff to view and trace.

It should be noted that the architecture of the system for detecting the ECG signal mentioned above is only one of preferable implementations, and it does not mean that only such architecture can serve as the implementation. In other embodiments, the application program corresponding to a detection method may be directly embedded into another electronic device for implementation. For example, the ECG machine 1 directly implements the analysis on the ECG signal without the user terminal 2, the server 3, and the local terminal 4. Alternatively, the ECG machine 1 may collect the ECG signal and the user terminal 2 may implement the analysis on the ECG signal, without the server 3 and the local terminal 4. Alternatively, the ECG machine 1 may collect the ECG signal and the local terminal 4 implement the analysis on the ECG signal, without the user terminal 2 and the server 3.

Hereinafter technical solutions in embodiments of the present disclosure are described clearly and completely in conjunction with the drawings in embodiments of the present disclosure. Apparently, the described embodiments are only some rather than all of the embodiments of the present disclosure. Any other embodiments obtained based on the embodiments of the present disclosure by those skilled in the art without any creative effort fall within the scope of protection of the present disclosure.

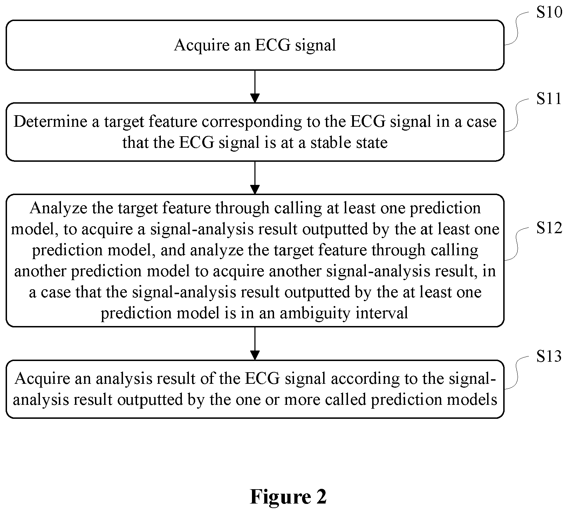

Figure 2 is a flow chart of a method for detecting an ECG signal according to an embodiment of the present disclosure. As shown in Figure 2, the method may be implemented based on a device storing a program for implementing this solution, such as a server or a mobile phone. Hereinafter the server is taken as an example. Sequential numbers of following steps are only intended to facilitate description, and it does not mean that the steps can only be implemented in such order. Different steps may be executed for a same quantity of times, or for different quantities of times. The method shown in Figure 2 includes steps S10 to S13.

In step S10, an ECG signal is acquired.

In a specific embodiment, the ECG signal is collected by the ECG machine. Since the collection consumes a certain period, the ECG signal acquired in this step is usually a signal containing multiple cycles. Different cycles of the ECG signal may be same or different, and all these cycles are from a same body under test.

The ECG signal may be acquired in real time. That is, the user terminal acquires the ECG signal collected by the ECG machine, and then uploads the signal to the server in real time. Alternatively, the ECG signal may not be acquired in real time. That is, the user terminal acquires a complete collection period of the ECG signal collected by the ECG machine, and then uploads the ECG signal in such period all together. Generally, the ECG signal received by the server has been processed by the user terminal or the host, in order to reduce a computing pressure of the server. Interference signals may be implemented via a hardware circuit. For example, the ECG signal is filtered via a filter circuit in the host. In a specific embodiment, the filter circuit performs a high-pass filtering, a low-pass filtering, or a band-pass filtering on the received ECG signal, to remove possible high-frequency interference, low-frequency interference, or power-frequency interference around 50 Hz, respectively. A cut-off frequency of a passband should ensure that the filtered signal retains, to the maximum extent, a proper feature reflecting the ECG signal. For example, a signal from 0.1Hz to 40Hz is retained.

In step S11, a target feature corresponding to the ECG signal is determined in a case that the ECG signal is at a stable state.

The ECG signal contains a lot of features for predicting whether the ECG signal is normal. The calculation efficiency would be greatly reduced in a case that every feature participates in a subsequent analysis. Therefore, it is necessary to determine the target feature corresponding to the ECG signal in this step. A type of the target feature is not limited herein. For example, the ECG signal may be analyzed in a time domain and a frequency domain. Amplitude, a parameter of amplitude variation, an energy spectral density, and a moment corresponding to the maximum amplitude, of each component wave in the ECG signal, may be extracted under a requirement on an optimal restore of the ECG component waves, and the above parameters serve as the target feature. In a specific embodiment, the component waves include a P wave, a QRS wave, a T wave, a U wave and a J wave. When extracting the target feature from each component wave in the ECG signal, it is necessary to ensure that the component waves of a same type are completely consistent with each other among different leads at corresponding moments.

Given that the amplitude of some component wave in some ECG signal is too small, it is necessary to transform the excessively small component wave independently into the frequency domain, so as to determine the amplitude thereof from the frequency domain. For example, the transform may adopt wavelet transform. Given that it is difficult to determine starting and ending points of some component wave of some ECG signal, it is necessary to calculate slopes of a left shoulder slope and a right shoulder of such component wave. A line crossing a midpoint of the left shoulder and having the slope of the left shoulder intersects with a base line at the starting point, and a line crossing a midpoint of the right shoulder and having the slope of the right shoulder intersects with the base line at the ending point.

In addition, before the target feature is extracted, the ECG signal may be subject to at least one algorithm of: Fourier transform, wavelet transform, or baseline-drift correction, to obtain a frequency spectrum of each component wave corresponding to the ECG signal, in a case that the component wave is not obvious in the time domain. Thus, the frequency domain can be presented more clearly, which facilitates the extraction of the target feature.

Determination of whether the ECG signal is at the stable state in this step may be implemented by the server or the the user terminal. In one solution, the server first acquires the ECG signal, and then determines the target feature corresponding to the ECG signal after determining that the ECG signal is at the stable state. In another solution, the user terminal first acquires the ECG signal, and then uploads the ECG signal to the server after determining that the ECG signal is at the stable state. In such case, the ECG signal acquired by the server is the stable signal, and the server determines the target feature corresponding to the ECG signal. Alternatively, the user terminal uploads the ECG signal upon acquisition, and further determines whether the ECG signal is at the stable state. The user terminal sends a stability identifier to the server when the ECG signal is at the stable state. Upon receiving the identifier, the server determines the target feature corresponding to the ECG signal that is at the stable state.

In summary, there is no strict sequence between the two steps S10 and S11, as long as the target feature corresponding to the ECG signal is determined after the ECG signal reaches the stable state. Thereby, accuracy of the detection result is improved.

In step S12, the target feature is analyzed through calling at least one prediction model, to acquire a signal-analysis result outputted by the at least one prediction model. The target feature is further analyzed through calling another prediction model to obtain another signal-analysis result, in a case that the signal-analysis result outputted by the at least one prediction model is in an ambiguity interval.

In a specific embodiment, the server stores prediction models, and a quantity of the models is not limited. Generally, there should be two or more models. The prediction model may be trained through samples that include ECG signals and corresponding clinical test results. In a specific embodiment, the samples are divided into training samples, test samples, and verification samples. The more the samples are, the more accurate the output result of the prediction model is. The prediction model may be acquired through a neural network or a support vector machine. In a specific embodiment, the neural network include: a convolutional neural networks (CNN), a long short-term memory network (LSTM), a recurrent neural networks (RNN), an extra trees classifier, a random forest classifier, a decision trees classifier, or the like.

In this embodiment, the quantity of the prediction models that are initially called is not limited, and may be one or more. In case of one prediction model, the target feature is analyzed through calling another prediction model to obtain another signal-analysis result, in a case that the signal-analysis result outputted by such model is in the ambiguity interval. In case of multiple prediction models, the condition "the signal-analysis result of the at least one prediction models is in the ambiguity interval" is satisfied as long as any signal-analysis result outputted by the called prediction models is in the ambiguity interval. In such case, it is necessary to analyze the target feature through calling another prediction model, so as to obtain another signal-analysis result.

The ambiguity interval in embodiments of the present disclosure means that an ECG signal falling in such interval has both a possibility of being normal and a possibility of being abnormal, and hence needs to be further identified. The ambiguity interval may be set as an interval within ±Δ (for example, Δ is equal to 5%×5mEq/L) at a threshold for distinguishing the normal signal and the abnormal signal. For example, a normal level of serum potassium is 5mEq/L, which is an indicator for the ECG signal being normal, and hyperkalemia is defined as being higher than 5mEq/L in the clinical standard. An error in the prediction model or another reason may result in an error in the signal-analysis result outputted by the prediction model. Thus, when the outputted signal-analysis result is within an interval of 5mEq/L±Δ, the signal-analysis result has both possibilities of being normal and being abnormal. In a case that the signal-analysis result outputted by the called prediction model(s) is in the ambiguity interval, another prediction model need to be called to analyze the target feature again. Herein the "another" prediction model is different from the previously called prediction model(s). That is, in this step, whether the outputted signal-analysis result is in the ambiguity interval determines whether more prediction model would participate in the analysis. It is appreciated that the more prediction models are called, the more effectively an error caused by the prediction model(s) is avoided, and the more accurate the acquired analysis result is.

In step S13, an analysis result of the ECG signal is acquired according to the signal-analysis result outputted by the one or more called prediction models.

The analysis result is at least configured to identify whether the ECG signal is normal or abnormal.

In the step S12, there may be one or more prediction models, and hence a quantity of the obtained signal-analysis results is at least one. In this step, a relationship between the signal-analysis result outputted by each prediction model and the final analysis result is not limited. As example, the signal-analysis result(s) outputted by the prediction model(s) called in the step S12 may not be in the ambiguity interval. In a case that only one prediction model is called, the signal-analysis result outputted by such model may serve as the final analysis result. In a case that multiple prediction models are called, the signal-analysis results outputted by the multiple called prediction models may be averaged or weighted to obtain the final analysis result. As another example, the signal-analysis result(s) outputted by the prediction model(s) called in step S12 is in the ambiguity interval. In such case, the signal-analysis results outputted by all the prediction models may be averaged or weighted to obtain the final analysis result. It is appreciated that when the number of called prediction models is relatively large, some abnormal result(s) may be removed according to a relationship among the signal-analysis results indicating normal or abnormal. For example, five prediction models are called, the signal-analysis results outputted by four prediction models indicate that the ECG signal is normal, and the signal-analysis result outputted by the remaining prediction model indicates that the ECG signal is abnormal. In such case, the signal-analysis result indicating abnormal may be removed, and the remaining four signal-analysis results are averaged to obtain the final analysis result.

In a specific embodiment, the method further includes a following step after the final analysis result is acquired, so as to facilitate users and operators to view. A waveform and the final analysis result of the ECG signal corresponding to the target feature are outputted. Correspondingly, the user can view the analysis result via the user terminal, and the operator can view the analysis result via the local terminal. In addition, the method further includes following steps. A biochemical detection result of the ECG signal corresponding to the target feature is acquired. The biochemical detection result is added as a test sample into original training samples, to supplement the original training samples. A new prediction model is acquired by training with the updated training samples. More training samples lead to adjusted parameters of the new prediction model in comparison with the original prediction model, thereby implementing self-learning. Moreover, the newly added sample is a biochemical detection result with higher accuracy, thereby rendering the new prediction model more accurate.

In other embodiments, the method further includes a following step. A notice corresponding to the analysis result is determined and outputted, such that a user and an operator can view the notice.

The method for detecting the ECG signal according to these embodiments includes following steps. The target feature corresponding to the ECG signal is determined in a case that the ECG signal is acquired and the ECG signal is at the stable state. The at least one prediction model is called to analyze the target feature, such that the signal-analysis result outputted by the called prediction model is acquired. The signal-analysis result is at least configured to identify whether the ECG signal is normal or abnormal. Hence, when applying such technical solution, stability is determined before the ECG signal is subject to actual prediction, which can eliminate an error in a calculating result due to signal instability. When the at least one prediction model is called to analyze the target feature, another prediction model is further called to analyze the target feature in a case that the signal-analysis result outputted by called prediction model(s) is in the ambiguity interval. Thereby, at least two prediction models can be applied to analyze the target feature in a case that reliability of the signal-analysis result outputted by a prediction model is low. Accuracy of the analysis is improved.

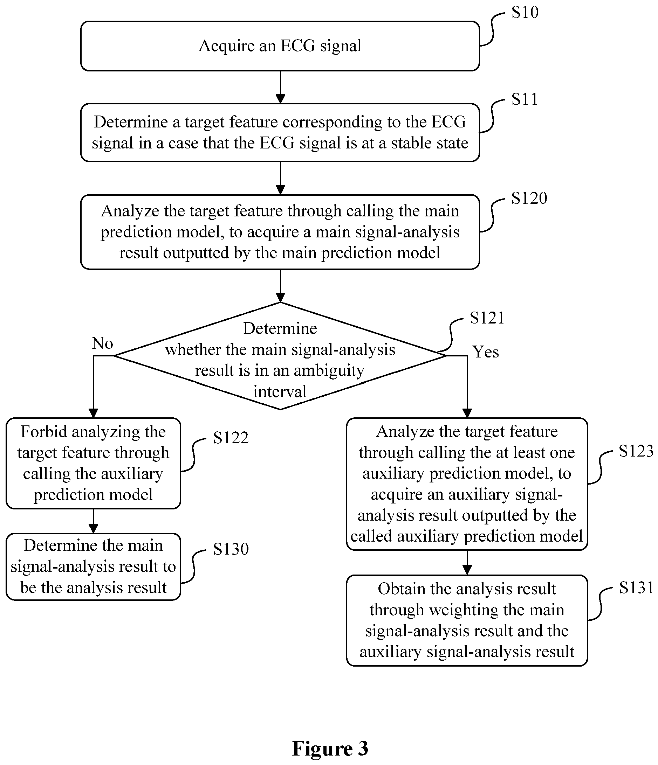

Figure 3 is a flow chart of another method for detecting an ECG signal according to an embodiment of the present disclosure. It can be appreciated that the more accurate a prediction model is, the more reliable the analysis result obtained by the prediction model is. Therefore, in an embodiment based on the foregoing embodiments, the prediction model includes a main prediction model and at least one auxiliary prediction model, and the main prediction model is more accurate than the auxiliary prediction model. On a basis of the foregoing embodiment, the step S12 includes steps S120 to S123.

In step S120, the target feature is analyzed through calling the main prediction model, to acquire a main signal-analysis result outputted by the main prediction model.

In step S121, it is determined whether the main signal-analysis result is in the ambiguity interval. The process goes to S 122 in case of negative determination, and goes to step S123 in case of positive determination.

In step S122, it is forbidden to analyze the target feature through calling the auxiliary prediction model. Then, the process goes to S130.

In step S123, the target feature is analyzed through calling the at least one auxiliary prediction model, to acquire an auxiliary signal-analysis result outputted by the called auxiliary prediction model.

It is appreciated that since the main prediction model is more accurate than the auxiliary prediction model, reliability of the signal-analysis result outputted by the main prediction model is higher. In a case that such result is not in the ambiguity interval, the result alone can serve as the final analysis result, and it is not necessary to further apply the auxiliary prediction model to analysis. Hence, efficiency of the prediction is improved. Similarly, in a case that such result is in the ambiguity interval, the result alone cannot serve as the final analysis result, and it is necessary to further apply the auxiliary prediction model to analysis. Hence, the accuracy of the analysis result is improved.

A quantity of the auxiliary prediction models in step S123 is not limited, and generally, there are more than two auxiliary prediction models.

In a preferable embodiment, the method further includes a following step in a case that the main signal-analysis result is in the ambiguity interval, so as to reduce an influence of the signal-analysis result being in the ambiguity interval. In a case that the auxiliary signal-analysis result is still in the ambiguity interval, an objective function of the main prediction model is adjusted, and the process return to the step S121.

In a preferable embodiment, the method further includes a following step in a case that the main signal-analysis result is in the ambiguity interval, so as to reduce an influence of the signal-analysis result being in the ambiguity interval. In a case that the auxiliary signal-analysis result is still in the ambiguity interval, a quantity of the called auxiliary prediction models is increased, and the process goes to step S123.

Further, the step S13 includes steps S130 to S131.

In step S130, the main signal-analysis result is determined to be the analysis result, in a case that the main signal-analysis result is not in the ambiguity interval.

In step S131, the analysis result is obtained through weighting the main signal-analysis result and the auxiliary signal-analysis result, in a case that the main signal-analysis result is in the ambiguity interval.

Since the main prediction model is highly accurate, the main signal-analysis result serves as the analysis result directly when being not in the ambiguity interval. Not only accuracy of the analysis result is ensured, but also an amount of calculation is reduced. Similarly, the auxiliary signal-analysis result is necessary when the main analysis result is in the ambiguity interval. Weighting the main analysis result and the secondary analysis result can prevent an error caused by a single model and ensure the accuracy of the analysis result.

In the foregoing embodiments, a manner for determining whether the ECG signal is at the stable state is not limited. In one embodiment, a following manner is adopted to determine whether the ECG signal is at the stable state.

One or both of fluctuations among peaks and fluctuations among valleys in one or more component waves of a same type are acquired from the ECG signal within N cycles. N is a positive integer.

It is determined that the ECG signal is at the stable state, in a case that the currently acquired fluctuations meet a preset condition.

It is determined that the ECG signal is not at the stable state, in a case that the currently acquired fluctuations do not meet the preset condition.

In a specific embodiment, N ranges from 5 to 10. In addition, the preset condition in this embodiment is determined according to the type of the currently acquired fluctuation. In a specific embodiment, the determination is as follows.
1) In a case that the currently acquired fluctuations are the fluctuations among the peaks of one or more component waves of the same type that are acquired from the ECG signal within N cycles, the preset condition is that any fluctuation among the peaks is less than a first preset threshold.
2) In a case that the currently acquired fluctuations are the fluctuations among the valleys of one or more component waves of the same type that are acquired from the ECG signal within N cycles, the preset condition is that any fluctuation among the valleys is less than a second preset threshold.
3) In a case that the currently acquired fluctuations are both the fluctuations among the peaks and the fluctuations among the valleys of one or more component waves of the same type that are acquired from the ECG signal within N cycles, the preset condition is that any fluctuation among the peaks is less than a first preset threshold and any fluctuation among the valleys is less than a second preset threshold.

The first preset threshold and the second preset threshold may be same or different. In a preferable embodiment, the currently acquired fluctuations are generally both the fluctuations among the peaks and the fluctuations among the valleys, so as to improve accuracy of determining the stability.

In a specific embodiment, the process of determining that the ECG signal is at the stable state may be implemented by the user terminal, so as to reduce the computing pressure of the server and further reduce transmission of useless signals. A buffer module is provided in the user terminal, and is configured to buffer the acquired ECG signal. In a specific embodiment, a sampling frequency may range from 200/sec to 2000/sec. The ECG signal is uploaded to the server in case of being at the stable state, and is not uploaded to the server in case of not being at the stable state. The buffering process adopts a manner of first-in-first-out (FIFO) circulating storage, to ensure that a signal in the buffer is always the most recently collected signal.

In another embodiment, a threshold for acquisition duration may be further set to avoid long-term invalid acquisition when the ECG signal is continuously instable. A prompt indicating the signal being unstable is outputted, in a case that the actual acquisition duration exceeds such threshold and the ECG signal has not reached the stable state. Accordingly, an operator is prompted to check a specific cause, such as restlessness of the body under test or detachment of an electrode. In a preferable embodiment, the threshold for acquisition duration is 30 seconds. Further, a corresponding solution may be determined according to the currently collected ECG signal. For example, if all component waves in the ECG signal are instable, the corresponding solution is to fix the body under test. In a specific embodiment, a manner of outputting the prompt of the signal being unstable is not limited. The manner may be presenting on a display interface of the local terminal and the user terminal, or turning on an indicator on the ECG machine.

In a specific implementation, the ECG signal contains many features. It is appreciated that the amount of calculation increases in a case that all features participate in the subsequent analysis. In view of the above, a target feature is selected in one embodiment. In Table 1, recall rates of different combinations of leads are compared (taking serum potassium as an example). It can be seen from Table 1 that the quadruple-lead combination shows the best recall rate. Experiments determine that signals of the leads V2 to V5 are selected from the 12-lead ECG signal, and the target feature is extracted from signals of the leads V2 to V5.

**Table 1**

| ECG signal Sample | Single-lead recall rate | Double-lead recall rate | Triple-lead recall rate | Quadruple-lead recall rate |
|---|---|---|---|---|
| 1 | 85.00% | 90.00% | 95.12% | 100.00% |
| 2 | 7.50% | 20.00% | 32.50% | 90.00% |
| 3 | 47.50% | 43.33% | 32.50% | 100.00% |
| 4 | 92.50% | 93.33% | 92.50% | 100.00% |
| 5 | 85.00% | 91.67% | 60.00% | 100.00% |
| 6 | 82.50% | 88.33% | 100.00% | 100.00% |
| 7 | 70.00% | 56.67% | 57.50% | 100.00% |
| 8 | 77.50% | 83.33% | 80.00% | 90.00% |
| 9 | 77.50% | 86.67% | 72.50% | 100.00% |
| 10 | 40.00% | 43.33% | 55.00% | 80.00% |
| 11 | 67.50% | 81.67% | 92.50% | 100.00% |
| 12 | 75.00% | 88.33% | 62.50% | 100.00% |
| 13 | 60.00% | 63.33% | 62.50% | 100.00% |
| 14 | 77.50% | 70.00% | 60.00% | 80.00% |
| 15 | 75.00% | 93.33% | 97.50% | 90.00% |
| 16 | 80.00% | 71.67% | 67.50% | 100.00% |
| 17 | 32.50% | 28.33% | 20.00% | 80.00% |
| 18 | 25.00% | 18.33% | 17.50% | 100.00% |
| 19 | 35.00% | 48.33% | 57.50% | 100.00% |
| 20 | 40.00% | 36.67% | 25.00% | 80.00% |
| 21 | 75.00% | 88.33% | 82.50% | 100.00% |
| 22 | 77.50% | 90.00% | 100.00% | 100.00% |
| 23 | 72.50% | 90.00% | 95.00% | 100.00% |
| 24 | 75.00% | 91.67% | 97.50% | 100.00% |
| 25 | 75.00% | 86.67% | 100.00% | 90.00% |

Although selected, the signals of the leads V2 to V5 still contain a lot of features. The features include amplitude of each component wave, a parameter of amplitude variation of each component wave, an energy spectral density of each component wave, and a moment corresponding to the maximum amplitude of each component wave, and the like. The ECG signal in each lead contains at least 16 features: a slope of a right shoulder of the T wave, amplitude of the T wave, a slope of a left shoulder of the T wave, a slop of the S-T segment, amplitude of the R wave, amplitude of the P wave, amplitude of the S wave, an area of the T wave, an area of the P wave, an area of the S wave, the QT interval, the QRS interval, the PR interval, a variation rate of unit areas of the T wave (per second), a variation rate of unit areas of the R wave (per second), and a variation rate of unit areas of the S wave (per second). In order to reduce the amount of calculation, extracting the target feature from the signals of leads V2 to V5 includes following steps.

Features are acquired from the ECG signals of the leads V2 to V5.

Heat values corresponding to the features are acquired.

The target feature is selected from the features based on the heat values.

In a specific embodiment, there are two manners of selecting the target feature from the features based on the heat values. In one manner, the features corresponding to the top M heat values among the sequenced heat values are selected as the target feature. In another manner, all heat values are compared with a heat threshold, and the feature(s) corresponding to the heat value(s) greater than the heat threshold are selected as the target feature.

The feature extraction with respect to each component wave is not performed on one lead, but on multiple leads. Among different leads, waveforms of the component waves of the same type are consistent with each other in moment features. For example, starting points of the R waves are consistent among the multiple leads. In a case that the R waves in the multiple leads have different starting points, repair processing such as interpolation, interval, and shifting may be applied to align their positions.

In the foregoing embodiments, the method for detecting the ECG signal has been described in detail, and embodiments corresponding to an apparatus for detecting an ECG signal is further provided according to the present disclosure. The present disclosure describes the apparatus embodiments based on two perspectives. One is a perspective of functional modules, and the other is a perspective of a hardware structure.

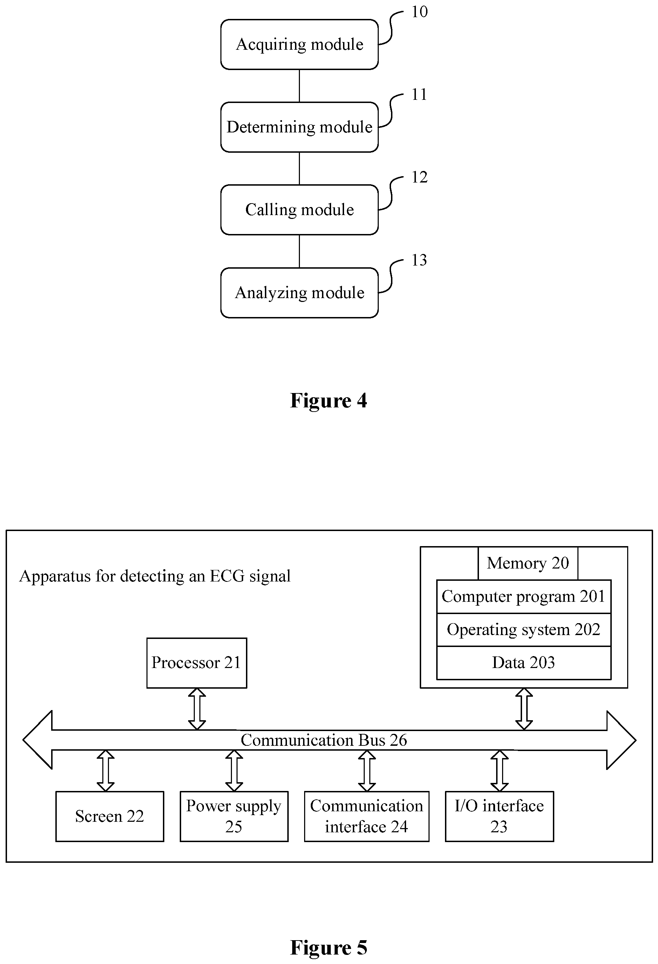

Reference is made to Figure 4, which is a schematic structural diagram of an apparatus for detecting an ECG signal according to an embodiment of the present disclosure. As shown in Figure 4, the apparatus includes an acquiring module 10, a determining module 11, a calling module 12, and an analyzing module 13.

The acquiring module 10 is configured to acquire an ECG signal.

The determining module 11 is configured to determine a target feature corresponding to the ECG signal in a case that the ECG signal is at a stable state.

The calling module 12 is configured to analyze the target feature through calling at least one prediction model, to acquire a signal-analysis result outputted by the called prediction model. The calling module 12 is further configured to analyze the target feature through calling another prediction model, to acquire another signal-analysis result, in a case that the signal-analysis result outputted by the called prediction model is in an ambiguity interval.

The analyzing module 13 is configured to acquire an analysis result of the ECG signal according to the signal-analysis result outputted by the one or more called prediction models. The analysis result is at least configured to identify whether the ECG signal is normal or abnormal.

In a preferable embodiment, the prediction model includes a main prediction model and at least one auxiliary prediction model. Accuracy of the main prediction model is higher than that of the auxiliary prediction model.

The calling module 12 includes a calling unit and a controlling unit.

The calling unit is configured to analyze the target feature through calling the main prediction model, to acquire a main signal-analysis result outputted by the main prediction model.

The controlling unit is configured to forbid analyzing the target feature through calling the auxiliary prediction model, in a case that the main signal-analysis result is not in the ambiguity interval. The controlling unit is further configured to analyze the target feature through calling the at least one auxiliary prediction model, to acquire an auxiliary signal-analysis result outputted by the called auxiliary prediction model, in a case that the main signal-analysis result is in the ambiguity interval.

As a preferable embodiment, the analyzing module 13 includes a first analyzing unit and a second analyzing unit.

The first analyzing unit is configured to determine the main signal-analysis result to be the analysis result, in a case that the main signal-analysis result is not in the ambiguity interval.

The second analyzing unit is configured to obtain the analysis result through weighting the main signal-analysis result and the auxiliary signal-analysis result, in a case that the main signal-analysis result is in the ambiguity interval.

As a preferable embodiment, the apparatus further includes a stability-determining module.

The stability-determining module is configured to acquire, from the ECG signal within N cycles, one or both of fluctuations among peaks and fluctuations among valleys in one or more component waves of a same type. N is a positive integer. The stability-determining module is further configured to determine that the ECG signal is at the stable state, in a case that the currently acquired fluctuations meet a preset condition. The stability-determining module is further configured to determine that the ECG signal is not at the stable state, in a case that the currently acquired fluctuations do not meet the preset condition.

As a preferable embodiment, the determining module 11 includes a first extracting unit and a second extracting unit.

The first extracting unit is configured to extract signals of leads V2 to V5 from the ECG signal.

The second extracting unit is configured to extract the target feature from the signals of leads V2 to V5.

As a preferable embodiment, the second extracting unit includes a first acquiring sub-unit, a second acquiring sub-unit, and a selecting sub-unit.

The first acquiring sub-unit is configured to acquire features from the signals of leads V2 to V5.

The second acquiring sub-unit is configured to acquire heat values corresponding to the features.

The selecting sub-unit is configured to select the target feature from the features based on the heat values.

The features include amplitude, a parameter of amplitude variation, an energy spectral density, and a moment corresponding to maximum amplitude, of each component wave.

Since the apparatus embodiments correspond to the method embodiments, the apparatus embodiments may refer to the description of the method embodiments, which is not repeated herein.

The apparatus for detecting the ECG signal is provided according to embodiments of the present disclosure. The target feature corresponding to the ECG signal is determined in a case that the ECG signal is acquired and the ECG signal is at the stable state. The at least one prediction model is called to analyze the target feature, such that the signal-analysis result outputted by the called prediction model is acquired. The signal-analysis result is at least configured to identify whether the ECG signal is normal or abnormal. Hence, when applying such technical solution, stability is determined before the ECG signal is subject to actual prediction, which can eliminate an error in a calculating result due to signal instability. When the at least one prediction model is called to analyze the target feature, another prediction model is further called to analyze the target feature in a case that the signal-analysis result outputted by called prediction model(s) is in the ambiguity interval. Thereby, at least two prediction models can be applied to analyze the target feature in a case that reliability of the signal-analysis result outputted by a prediction model is low. Accuracy of the analysis is improved.

Reference is made to Figure 5, which is a schematic structural diagram of another apparatus for detecting an ECG signal according to an embodiment of the present disclosure. As shown in Figure 5, the apparatus includes a memory 20 and a processor 21. The memory 20 is configured to store a computer program,.

The processor 21 is configured to implement the aforementioned method when executing the computer program.

The apparatus for detecting the ECG signal in this embodiment may include, but is not limited to, a smart phone, a tablet computer, a laptop computer, or a desktop computer.

The processor 21 may include one or more processing cores, such as a four-core processor, an eight-core processor, and so on. The processor 21 may be realized by adopting at least one hardware form of DSP (digital signal processing), an FPGA (field-programmable gate array), or a PLA (programmable logic array). The processor 21 may further include a main processor and a co-processor. The main processor is a processor configured to process data in an awake state, and is also called a CPU (central processing unit). The co-processor is a low-power processor configured to process data in a standby state. In some embodiments, the processor 21 may further be integrated with a GPU (graphics processing unit), which is configured to render and draw content that needs to be displayed on a screen. In some embodiments, the processor 21 may further include an AI (artificial intelligence) processor, which is configured to process calculating operations related to machine learning.

The memory 20 may include one or more computer-readable storage media, and may be non-transitory. The memory 20 may also include a high-speed random access memory and a non-volatile memory, such as one or more magnetic disk devices and flash memory devices. In this embodiment, the memory 20 is configured to store at least a following computer program 201, and the computer program when loaded and executed by the processor 21 is capable to implement relevant steps of the method for detecting the ECG signal in any foregoing embodiments. In addition, the resources stored in the memory 20 may further include an operating system 202, data 203, and the like, and the storage may be short-term or permanent. The operating system 202 may include Windows, Unix, Linux, or the like. The data 203 may include, but is not limited to, the ECG signal or the like.

In some embodiments, the apparatus for detecting the ECG signal may further include a screen 22, an input/output interface 23, a communication interface 24, a power supply 25, and a communication bus 26.

Those skilled in the art can understand that the apparatus for detecting ECG signal is not limited to the structure as shown in Figure 5, and may include more or fewer components than those shown in the figure.

The apparatus for detecting the ECG signal in embodiments of the present disclosure includes a memory and a processor. The processor when executing a program stored in the memory is capable to implement a following method. A target feature corresponding to an ECG signal is determined in a case that the ECG signal is acquired and the ECG signal is at a stable state. At least one prediction model is called to analyze the target feature, such that a signal-analysis result outputted by the called prediction model is acquired. The signal-analysis result is at least configured to identify whether the ECG signal is normal or abnormal. Hence, when applying such technical solution, stability is determined before the ECG signal is subject to actual prediction, which can eliminate an error in a calculating result due to signal instability. When the at least one prediction model is called to analyze the target feature, another prediction model is further called to analyze the target feature in a case that the signal-analysis result outputted by called prediction model(s) is in the ambiguity interval. Thereby, at least two prediction models can be applied to analyze the target feature in a case that reliability of the signal-analysis result outputted by a prediction model is low. Accuracy of the analysis is improved.

The present disclosure further provides an embodiment corresponding to a computer-readable storage medium. The computer-readable storage medium stores a computer program, and the computer program when executed by the processor implements steps described in the forgoing method embodiments.

In a case that the method in the forgoing embodiments is implemented as a software functional unit and is sold or used as an independent product, the method may be stored in a computer readable storage medium. Based on such understanding, parts of the essence of the technical solution or that contributes to the prior art, or all or parts of the technical solution may be embodied in the form of a software product. The software product is stored in the storage medium to execute all or parts of the steps of the method described in each embodiment of the present disclosure. The storage media includes: an USB flash drive, a portable hard drive, a read-only memory (ROM), a random access memory (RAM), a magnetic disk or optical disk and other media that can store program codes.

The method and apparatus for detecting the ECG signal provided by the present disclosure are described in detail above. The embodiments of the present disclosure are described in a progressive manner, and each embodiment places emphasis on the difference from other embodiments. Therefore, one embodiment may refer to other embodiments for the same or similar parts. For the apparatuses disclosed in the embodiments, since they correspond to the methods disclosed in the embodiments, the description is relatively simple, and the relevant parts may refer to the description of the methods. It should be pointed out that those skilled in the art can make several improvements and modifications to the present disclosure without departing from the principles of the present disclosure, and these improvements and modifications also fall within the protection scope of the claims of the present disclosure.

It should be noted that, the relationship terms such as "first", "second" and the like are only used herein to distinguish one entity or operation from another, rather than to necessitate or imply that an actual relationship or order exists between the entities or operations. Furthermore, the terms such as "include", "comprise" or any other variants thereof means to be non-exclusive. Therefore, a process, a method, an article or a device including a series of elements include not only the disclosed elements but also other elements that are not clearly enumerated, or further include inherent elements of the process, the method, the article or the device. Unless expressively limited, the statement "including a..." does not exclude the case that other similar elements may exist in the process, the method, the article or the device other than enumerated elements.

## Claims

1. A method for detecting an electrocardiogram (ECG) signal, comprising:
acquiring an ECG signal;
determining a target feature corresponding to the ECG signal in a case that the ECG signal is at a stable state;
analyzing the target feature through calling at least one prediction model, to acquire a signal-analysis result outputted by the at least one prediction model;
analyzing the target feature through calling another prediction model to acquire another signal-analysis result, in a case that the signal-analysis result outputted by the at least one prediction model is in an ambiguity interval; and
acquiring an analysis result of the ECG signal according to the signal-analysis result outputted by the one or more called prediction models, wherein the analysis result is at least configured to identify whether the ECG signal is normal or abnormal.

2. The method according to claim 1, wherein the prediction models comprises a main prediction model and at least one auxiliary prediction model, the main prediction model is more accurate than the auxiliary prediction model, and
analyzing the target feature through calling at least one prediction model, to acquire the signal-analysis result outputted by the at least one prediction model, and analyzing the target feature through calling another prediction model to acquire another signal-analysis result, in the case that the signal-analysis result outputted by the at least one prediction model is in the ambiguity interval, comprises:
analyzing the target feature through calling the main prediction model, to acquire a main signal-analysis result outputted by the main prediction model;
forbidding analyzing the target feature through calling the auxiliary prediction model, in a case that the main signal-analysis result is not in the ambiguity interval; and
analyzing the target feature through calling the at least one auxiliary prediction model, to acquire an auxiliary signal-analysis result outputted by the at least one auxiliary prediction model, in a case that the main signal-analysis result is in the ambiguity interval.

3. The method according to claim 2, wherein acquiring the analysis result of the ECG signal according to the signal-analysis result outputted by the one or more called prediction models, comprises:
determining the main signal-analysis result to be the analysis result, in a case that the main signal-analysis result is not in the ambiguity interval; and
obtaining the analysis result through weighting the main signal-analysis result and the auxiliary signal-analysis result, in a case that the main signal-analysis result is in the ambiguity interval.

4. The method according to any one of claims 1 to 3, wherein whether the ECG signal is at the stable state is determined by:
acquiring, from the ECG signal within N cycles, one or both of fluctuations among peaks and fluctuations among valleys in one or more component waves of a same type, wherein N is a positive integer;
determining that the ECG signal is at the stable state, in a case that the currently acquired fluctuations meet a preset condition; and
determining that the ECG signal is not at the stable state, in a case that the currently acquired fluctuations do not meet the preset condition.

5. The method according to any one of claims 1 to 3, wherein determining the target feature corresponding to the ECG signal comprises:
extracting signals of leads V2 to V5 from the ECG signal; and
extracting the target feature from the signals of leads V2 to V5.

6. The method according to claim 5, wherein extracting the target feature from the signals of leads V2 to V5 comprises:
acquiring features from the signals of leads V2 to V5;
acquiring heat values corresponding to the features; and
selecting the target feature from the features based on the heat values;
wherein the features comprise amplitude, a parameter of amplitude variation, an energy spectral density, and a moment corresponding to maximum amplitude, of each component wave.

7. An apparatus for detecting an electrocardiogram (ECG) signal, comprising:
an acquiring module, configured to acquire an ECG signal;
a determining module, configured to determine a target feature corresponding to the ECG signal in a case that the ECG signal is at a stable state;
a calling module, configured to:
analyze the target feature through calling at least one prediction model, to acquire a signal-analysis result outputted by the at least one prediction model; and
analyze the target feature through calling another prediction model to acquire another signal-analysis result, in a case that the signal-analysis result outputted by the at least one prediction model is in an ambiguity interval; and
an analyzing module, configured to acquire an analysis result of the ECG signal according to the signal-analysis result outputted by the one or more called prediction models, wherein the analysis result is at least configured to identify whether the ECG signal is normal or abnormal.

8. The apparatus according to claim 7, wherein the prediction model comprises a main prediction model and at least one auxiliary prediction model, the main prediction model is more accurate than the auxiliary prediction model, and
the calling module comprises:
a calling unit, configured to analyze the target feature through calling the main prediction model, to acquire a main signal-analysis result outputted by the main prediction model;
a controlling unit, configured to:
forbid analyzing the target feature through calling the auxiliary prediction model, in a case that the main signal-analysis result is not in the ambiguity interval; and
analyze the target feature through calling the at least one auxiliary prediction model, to acquire an auxiliary signal-analysis result outputted by the at least one auxiliary prediction model, in a case that the main signal-analysis result is in the ambiguity interval.

9. The apparatus according to claim 8, wherein the analyzing module comprises:
a first analyzing unit, configured to determine the main signal-analysis result to be the analysis result, in a case that the main signal-analysis result is not in the ambiguity interval, and
a second analyzing unit, configured to obtain the analysis result through weighting the main signal-analysis result and the auxiliary signal-analysis result, in a case that the main signal-analysis result is in the ambiguity interval.

10. The apparatus according to any one of claims 7 to 9, further comprising a stability-determining module, configured to:
acquire, from the ECG signal within N cycles, one or both of fluctuations among peaks and fluctuations among valleys in one or more component waves of a same type, wherein N is a positive integer;
determine that the ECG signal is at the stable state, in a case that the currently acquired fluctuations meet a preset condition; and
determine that the ECG signal is not at the stable state, in a case that the currently acquired fluctuations do not meet the preset condition.

11. The apparatus according to any one of claims 7 to 9, wherein the determining module comprises:
a first extracting unit, configured to extract signals of leads V2 to V5 from the ECG signal; and
a second extracting unit, configured to extract the target feature from the signals of leads V2 to V5.

12. The apparatus according to claim 11, wherein the second extracting unit comprises:
a first acquiring sub-unit, configured to acquire features from the signals of leads V2 to V5;
a second acquiring sub-unit, configured to acquire heat values corresponding to the features; and
a selecting sub-unit, configured to select the target feature from the features based on the heat values;
wherein the features comprise amplitude, a parameter of amplitude variation, an energy spectral density, and a moment corresponding to maximum amplitude, of each component wave.
